(19) Europäisches Patentamt
European Patent Office
Office européen des brevets

(11) Publication number: **0 492 807 A2**

# (12) EUROPEAN PATENT APPLICATION

(21) Application number: **91310876.7**

(22) Date of filing: **26.11.91**

(51) Int. Cl.5: **C07C 45/53**, C07C 49/08, C07C 37/08, C07C 39/04, B01J 29/08

(30) Priority: **27.12.90 US 634629**

(43) Date of publication of application: **01.07.92 Bulletin 92/27**

(84) Designated Contracting States: **DE FR GB**

(71) Applicant: **TEXACO CHEMICAL COMPANY**
**3040 Post Oak Boulevard**
**Houston, Texas 77056(US)**

(72) Inventor: **Knifton, John Frederick**
**10900 Catskill Trail**
**Austin, Texas 78750(US)**
Inventor: **Dai, Pei-Shing Eugene**
**3437 Brittany**
**Port Arthur, Texas 77640(US)**

(74) Representative: **Green, Mark Charles et al**
**Urquhart-Dykes & Lord, 91 Wimpole Street**
**London W1M 8AH(GB)**

## (54) Method for production of phenol/acetone from cumene hydroperoxide.

(57) A process is disclosed for the synthesis of phenol and acetone by decomposition over a catalyst comprising solid acidic zeolite and subsequently isolating the product by fractional distillation. The method allows for the generation of phenol in selectivities as high as 99% or greater.

EP 0 492 807 A2

This invention relates to a method for decomposition of organic hydroperoxides and more particularly, this invention relates to a method for producing phenol and acetone by decomposition of cumene hydroperoxide over a definable class of solid acidic zeolite catalysts. The invention is particularly advantageous in that phenol is generated in selectivities as high as 99% mole.

It is known to those skilled in the art that cumene can be oxidized to cumene hydroperoxide and that cumene hydroperoxide can be decomposed by various means to provide phenol and acetone.

In the past certain acid catalysts have been used for producing phenol and acetone. Often the decomposition of cumene hydroperoxide is carried out in the presence of $H_2SO_4$. This process leads to the formation of a mixture including phenol, acetone, acetophenone and sulphonated by-products, the separation of which has a considerable effect on plant complexity and production costs. In the cases where acidic substances are utilized as the catalysts the yields are satisfactory, however many of these acid catalysts require substantial expenditure for production of phenol and acetone, there are disposal problems with spent acids or their salts and there are difficulties in achieving >99.9% purity phenol required by today's market place due to entrainment or breakthrough of said acids. In addition, by-products such as mesityl oxide, $\alpha$-methylstyrene, acetophenone and 2-phenyl-2-propanol are produced along with the product and must somehow be removed and processed.

U. S. Patent No. 4,898,995 discloses the decomposition of cumene hydroperoxide over a heterogeneous catalyst on an inert support or an ion exchange resin with a sulfonic acid functionality to synthesize phenol and acetone.

U. S. Patent No. 4,898,987 discloses the use of a catalyst comprising an acidic montmorillonite silica-alumina clay, modified with a compound from the group consisting of a heteropoly acid or the inorganic salt of zirconium, titanium or aluminium.

European Patent No. 0 203 632 discloses a method for decomposing cumene hydroperoxide over a catalyst comprising a zeolite crystal having a portion of the silicon atoms in the crystal lattice of silica replaced by A1 and B, wherein the zeolite crystals are bonded to each other by a siliceous bonding agent which allows the catalyst to assume the shape of mechanically stable microspheres. Phenol selectivities are typically 80-96% in the cited examples.

European Patent Applications 0 125 065 and 0 125 066 disclose the production of phenol and acetone by cleavage of cumene hydroperoxide in the presence of a solid heterogeneous catalyst with acidic activity comprising either an intermediate pore size zeolite, such as ZSM-5, or comprising zeolite beta. Application No. 0 125 065 also includes data for zeolite-Y, but acetone-phenol selectivities are only 59-78%, and the conversions are limited to 4-31% in these examples.

The use of zeolites to synthesize specified chemicals is known. For example, in an article entitled "Molecular Shape Selective Catalysis", by Weisz, P.B. in New Horizons in Catalysis, Part A, 3(1980) there is a discussion of molecular shape selective catalysis in intracrystalline spaces of siliceous zeolites. This article includes a table summarizing the major shape selective conversion processes which have been developed. There is no mention of the decomposition of cumene hydroperoxide.

For additional information on shape selective reactions, there is an article entitled "Shape Selective Reactions with Zeolite Catalysts", by L. B. Young et al., J. Catal 76 418(1982). The main focus of this work was presentation of data for para-selective and non-selective formation of xylenes from alkylation of toluene with methanol and toluene disproportionation over ZSM-5 class zeolite catalysts. It is noted that distinctly different reaction pathways are observed for the para-selective in comparison to non-selective and that this is probably a result of diffusional effects possibly superimposed on the sterically altered intrinsic kinetics.

US-A-4490565 describes the production of phenol and acetone by the cleavage of cumene hydroperoxide in the presence of a solid heterogeneous catalyst with acidic activity comprising zeolite beta. This has a characteristic structural framework.

US-A-4490566 describes the production of phenol and acetone by the cleavage of cumene hydroperoxide in the presence of a solid heterogeneous catalyst with acidic activity comprising an intermediate pore size zeolite such as ZSM-5.

US-A-4849387 describes a catalyst for the selective decomposition of cumene hydroperoxide comprised of zeolite crystals wherein a portion of the silicon atoms in the crystal is replaced by A1 and B.

Those skilled in the art in this field are continually searching for improved methods of producing phenol and acetone. It would be a substantial advance in the art if phenol and acetone could be produced in yields approaching 100% by decomposition over a durable catalyst using mild conditions. Furthermore a very active, long life catalyst would solve problems typically encountered with catalyst disposal and acid entrainment.

The present invention provides a novel method of continuous cogeneration of phenol and acetone comprising the steps of reacting cumene hydroperoxide in the presence of a catalyst which comprises one

or more solid acidic zeolites and optionally using a recycle loop continuous reactor design.

A particular advantage of the present invention over previous methods is that problems previously encountered involving $SO_3$ are ameliorated. There are no disposal problems; $SO_3$ or $H_2SO_4$ breakthroughs and corrosion are not problems and a greatly improved yield is observed. Every 1% yield improvement interprets into substantial cost savings in a typical plant operation. The method of the present invention allows for quantitative conversions with yields of up to 99 mole% or better.

For a better understanding of the present invention and to show how it may be put into effect, reference will now be made to the accompanying drawing in which:-

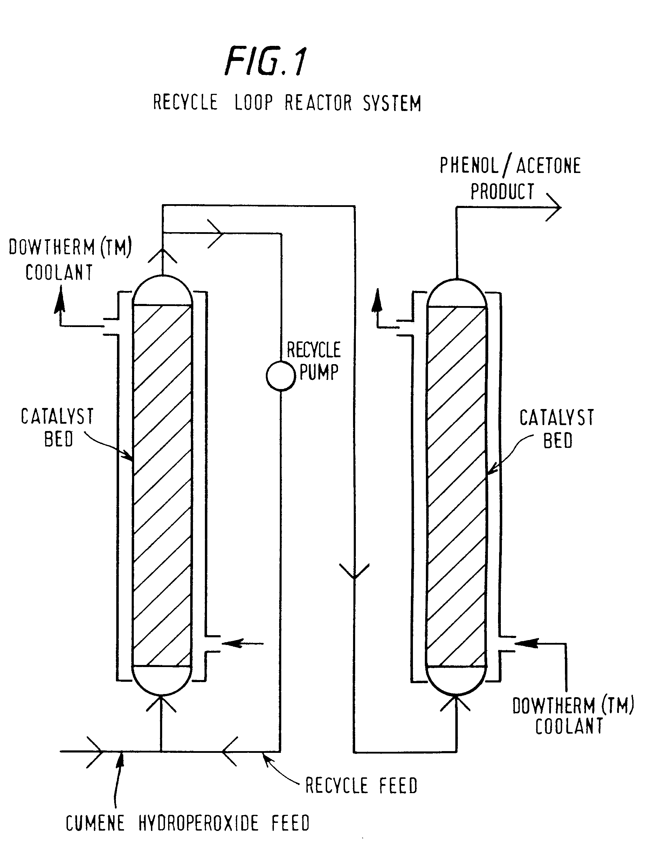

Figure 1 is a schematic illustration of the preferred process of the invention using a block diagram layout.

The process of this invention may be carried out typically by reacting cumene hydroperoxide continuously in the presence of a decomposition catalyst comprising a solid acidic zeolite. The catalyst is preferably employed as a series of solid acidic zeolites in a recycle loop reactor connected to a finishing reactor. The catalyst may be in tableted, granular or extruded form.

The reaction can be represented by the following:

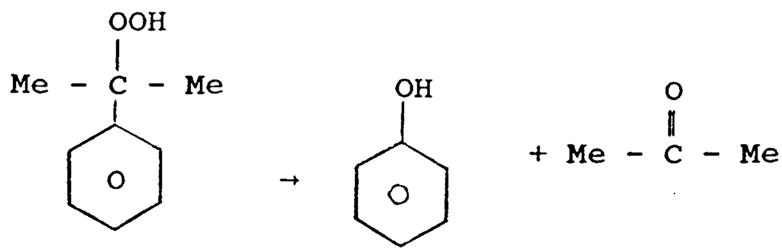

(Eq. 1)

The same method may also be applied to the reaction of other hydroperoxides. For example, the process may be applied to the decomposition of aromatic hydroperoxides such as secbutylbenzene hydroperoxide, ethylbenzene hydroperoxide and cyclohexylbenzene hydroperoxide.

The structures used as the catalysts to effect this reaction are acidic zeolites, particularly the isostructural group of faujasite zeolites that include the synthetic X and Y zeolites, the rare mineral faujasite and a number of other synthetic zeolites (see D. W. Beck, "Zeolite Molecular Sieves", Willey Interscience, 1974). The unit cells of faujasite and zeolites X and Y are cubic, $a_o \approx 2.5$ nm, and each contains 192 silicon- or aluminium-centred oxygen tetrahedra which are linked through shared oxygen atoms. Because of the net negative charge on each of the aluminium-centred tetrahedra, each unit cell contains an equivalent number of charge-balancing cations. These are exclusively sodium ions in zeolites X and Y in their synthesized form and a complex distribution between sodium, potassium, magnesium and calcium in naturally-occurring faujasite. Typical cell contents for the three zeolites in the hydrated form are:

faujasite $(Na_2,K_2Mg,Ca)_{29.5}[(A10_2)_{59}(SiO_2)_{133}],235H_2O$
zeolite X $Na_{86}[(A10_2)_{86}(SiO_2)_{106}],264H_2O$
zeolite Y $Na_{56}[A10_2)_{56}(SiO_2)_{136}],250H_2O$

Zeolites X and Y are distinguished on the basis of the relative concentration of silicon and aluminum atoms and the consequent effects on detailed structure and related chemical and physical properties. The aluminum atoms in the unit cell of zeolite X vary from 96 to 77 giving a Si:Al ratio between 1 and 1.5, whereas for zeolite Y they vary from 76 to 48 giving a Si:Al ratio between 1.5 and 3.0. It follows that both the cation concentration and charge density on the alumino-silicate structure are higher for X zeolite than for Y zeolite.

The feature which determines the difference between faujasites and other zeolites built up from sodalite units is the double 6-membered ring or hexagonal prism, by which the units are linked. The sodalite unit, or $\beta$-cage, can be represented by a truncated octahedrin, with the 24 silicon or aluminum atoms (designated T atoms) taking position at the vertices. The 36 oxygen atoms are displaced from the midpoints of the edges joining the vertices in order to attain tetrahedral configuration around the T atoms. The free diameter of the void within the $\beta$-cage is 0.66 nm, but only the smallest molecules can enter through the 0.22 nm diameter opening in the distorted ring of six oxygen atoms associated with each hexagonal face. Each sodalite unit is linked tetrahedrally across hexagonal faces by six bridging oxygens to four other sodalite units. The larger

void spaces enclosed by sodalite units and hexagonal prisms are termed α-cages, or supercages. The α-cage is a 26-hedron with a free diameter of ≈1.3 nm, and it can be entered through four distorted 12-member rings of diameter 0.80-0.90 nm. In this way each α-cage is tetrahedrally joined to four others giving a complex system of void space extending throughout the zeolite structure. The α- and β-cages together give X and Y zeolites the largest void volume of any known zeolite, which is ca. 50 vol% of the dehydrated crystal. From the catalytic viewpoint, however, the α-cages are by far the most important, since, unlike the β-cages, they permit entry of numerous aliphatic and aromatic compounds.

Particularly effective in the subject synthesis of phenol/acetone are the synthetic Y-zeolites. Preferably said zeolites should be in a strongly acidic form whereby some, or all, of the cations (Group I or II, alkali or alkaline earth metal ions such as sodium, potassium, calcium or magnesium) are exchanged by protons either through ammonium exchange followed by thermal stabilization (deammoniation, removal of $NH_3$) at elevated temperatures (e.g. 400-500 °C), through mineral acid treatment, etc. Alternatively, said Y-zeolites may be dealuminated by treatment with fluorine or fluoride ion, by hydrothermal treatment, by mineral acid treatment, by the combined hydrothermal and acid treatment, or by treatment with ethylenediaminetetraacetic acid (EDTA) or other chelating agents, in which case said dealuminized Y-zeolites should have a Si:A1 ratio of greater than three. A further possibility is that said Y-zeolites may be rare-earth exchanged with, for example, a mixture of rare-earth salts, by treatment with lanthanum salts, etc. Said rare-earth exchanged Y-zeolites would then have a Si:A1 ratio of 1.5 to 3. The exchange of the sodium ions of the Y-zeolite by rare earth, ammonium or alkylammonium ions has been reviewed (see, for example, R. Rudham and A. Stockwell, The Chemical Society Specialist Periodical Report - Catalysis, Vol. I, 1977 Chapter 3).

Dealuminated Y-zeolite may typically be prepared by treatment with ammonium fluoride for a period of time (e.g. 3-6 hours) at elevated temperatures (40-100 °C) in an aqueous media, followed by calcination at temperatures in the range of 400-600 °C.

A further possibility is the use of Y-zeolites in the subject application that have been modified by transition-metal exchange or impregnation, in particular, the use of Y-zeolites that have been modified by exchange of sodium ions etc., or by impregnation, of Group VIII metal salts such as those of cobalt(II) and nickel(II). Both methods are discussed in detail in an article by J.W. Ward, Applied Industrial Catalysis, Vol. 3, p. 271 (1984).

Said Y-zeolite, or modified Y-zeolites, may be employed alone, or to ensure greater physical strength and stability when in the form of extrudates, pellets, or granules, etc., they may also be used in the presence of certain binders. Suitable binders in the phenol/acetone application include silica-alumina binders, alumina binders and carbon binders, etc.

The zeolites useful in the instant invention are acidic solids, having titratable acidity in the range of between zero and 1.0 meq/g. or greater. The surface area of said Y-zeolites are generally greater than 400 $m^2/g$.

Illustrative of suitable zeolites for phenol/acetone generation from cumene hydroperoxide include typical Y-type zeolites, particularly those having a high silica to alumina ratio, i.e. a silica to alumina ratio greater than 5:1. More preferably, the super-dealuminated Y-zeolite has a silica to alumina ratio greater than about 10:1. Examples of suitable commercially available catalysts are PQ Corporation's ammonium-exchanged, CP304-37, having a silica to alumina ratio of about 11:1, CP316-26 having a silica to alumina ratio of 46, rare-earth exchanged zeolites such as the Linde SK-500 extrudates, having a Si:Al ratio of between 1.5:1 and 2:1, and produced by the Linde Division of Union Carbide, as well as Universal Oil Products LZ-10 that has been lanthanum ion exchanged. Also effective are ammonium exchanged Na-Y zeolites, having a sodium content of <0.5% and a silica:alumina ratio of 3:1, such as LZY-82, available from Union Carbide Corporation, together with ammonium fluoride treated Y-85 (from UOP).

Other zeolites useful in the practice of this invention are beta zeolites. The acidic properties of zeolite beta have been discussed (see E. Bourgeat-Lami et al., Catal. Lett., 265 (1990)) and, as a hybrid of two distinct but related structures, the unique features of the structure of zeolite beta have recently been enumerated by J. M. Newsman, et al., Chem. Eng. News, 66 (25), 22 (1988). Suitable beta zeolites for the practice of this invention include Valfor C861β, manufactured by PQ Corporation.

Mordonite zeolites are also effective for phenol/acetone generation from cumene hydroperoxides. The idealized composition of sodium mordonite is:

$$Na_8(AlO_2)_8(SiO_2)_{40}24H_2O$$

The structure consists of chains of tetrahedra cross-linked by the sharing of oxygen atoms. Each tetrahedra belongs to one or more five-membered rings in the framework. Unlike X and Y zeolite, the

mordonite structure contains no large supercages. The dehydrated structure has a two-dimensional channel system accessible to small molecules and a one-dimensional pore system of about 6.7A diameter, accessible to larger molecules.

Suitable mordonite zeolites include Zeolon 900M, having a $SiO_2/Al_2O_3$ ratio of 10-13, and available from the Norton Company or PQ Corporation.

The cumene hydroperoxide decomposition may be conducted batchwise, in a continuous slurry bed reactor, or in a fixed-bed, continuous flow, reactor. For practical reasons a fixed bed process is preferred. In Examples 1 through 18, the effectiveness of various acidic zeolites was tested using a recycled loop reactor connected in series with a finishing reactor.

Cogeneration of phenol and acetone can generally be conducted at temperatures from 20° to 150°C; the preferred range is 40° to 120°C. The operating pressure may be from zero to 6900 kPa gauge, or higher. The preferred pressure range is 100 to 2760 kPa gauge. Because of the highly exothermic nature (220 kJ/mole) of the cumene hydroperoxide decomposition (Eq. 1), temperature control is particularly important, especially in a fixed catalyst bed process.

Typically, phenol is generated continuously in up to ca. 60 wt% concentration in the crude product liquid effluent, and likewise, acetone may be generated in up to 40 wt% concentrations. The cumene hydroperoxide should preferably be as pure as possible, but 80% purity is certainly acceptable. Typical impurities in such an 80% cumene hydroperoxide feed are cumene, 2-phenyl-2-propanol and acetophenone. Optionally said cumene hydroperoxide may be diluted with inert solvent, or product, prior to being fed to the decomposer. Typical diluents include cumene, acetone, or a mix of acetone, cumene and phenol.

Generally cumene hydroperoxide conversions are quantitative in continuous unit operations. Phenol yields, based on hydroperoxide charged, are many times 99 mole% or better. Likewise, acetone yields are 95 mole% or better.

These yields are achieved at total liquid hourly space velocities (LHSV) of one to 10 under mild conditions. LHSVs of 10, or greater, have also been demonstrated to be useful in achieving quantitative cumene hydroperoxide conversion.

Here LHSV is defined as follows:

$$\text{LHSV} = \frac{\text{Weight Of Total Liquid Feed Run Through The Reactor Per Hour}}{\text{Volume of Catalyst In Reactor}}$$

The examples which follow illustrate the cogeneration of phenol and acetone from cumene hydroperoxide using solid acidic zeolite catalysts.

Conversion of cumene hydroperoxide (wt%) is estimated in the following examples using the equation:

$$\frac{\text{Wt\% Conc. of } (C_6H_5C(CH_3)_2OOH \text{ in Feed - Wt\% Conc.of Cumene Hydroperoxide in product]}}{\text{Wt\% Conc. of Cumene Hydroperoxide in Feed}} \times 100$$

Selectivities of phenol/acetone ($C_6H_5OH/CH_3COCH_3$, mole%) are estimated from:

$$\frac{\text{Moles of Phenol or Acetone in Product Liquid}}{\text{Moles of Cumene Hydroperoxide Consumed}} \times 100$$

The data in the attached Examples illustrate the conversion of cumene hydroperoxide to phenol/acetone catalyzed by a series of solid acid zeolites. Reaction is conducted continuously, under relatively mild conditions, using a loop reactor design (See Fig. 1).

Of note here:

In Example 1, using the SK-500 acidic zeolite catalyst, phenol is generated in >99 mole% and 96.9 mole% selectivities, basis glc and distillation data respectively. Acetone selectivity is excellent and cumene hydroperoxide conversion is quantitative, or near quantitative.

In Example 2-18, a series of additional solid acid zeolites have been evaluated and good cumene hydroperoxide conversions and good phenol selectivities have been realized with:

A) Ammonium exchanged Y-zeolites having a silica to alumina ratio of less than 10:1, such as Union

Carbide's LZY-82 (see Example 5).

B) Ammonium exchanged Y-zeolites having a silica to alumina ratio of greater than 10:1, such as PQ Corporations' CP304-37 (see Example 3) and CP316-26 (see Example 11).

C) Rare earth exchanged Y-zeolites such as SK-500,marketed by the Linde Division of Union Carbide (see Example 1), and lanthanum exchanged Y-zeolites such as La exchanged LZ-20 from United Oil Products (see Example 12).

D) Mordonite-type zeolites such as Zeolon-900H, having a silica to alumina ratio of 10-13:1 and marketed by PQ Corporation (see Example 4) and the Norton Company (see Example 8).

E) Beta Zeolites having a 25:1, $SiO_2/Al_2O_3$ ratio, such as Valfor C861$\beta$ (see Example 9).

## EXAMPLE 1

### Phenol/Acetone Generation Using Acidic Zeolite Catalyst

To a 150 cc capacity, recycle loop reactor system with heating/cooling capabilities which is connected in series with a 150 cc capacity finishing reactor (See Fig. 1), was charged a total of 300 cc of acidic zeolite (SK-500 from the Linde Division of Union Carbide, 0.26 meq/g titratable acidity, silica to alumina ratio 1.5-2, 10-20% alumina binder, 0.16cm diameter extrudates). Each reactor was operated upflow. A feed stream (0.43 kg/hr) of crude cumene hydroperoxide (sample FS, 75.1% by titration) diluted with cumene, was passed through both catalyst beds, which were maintained at a set temperature by circulating fluid in the reactor jackets. Reactor pressure was maintained at 2100 kPa, the flow of liquid in the recycle loop was set at 367 cc/min.

The liquid product effluent was collected, analyzed by glc, and samples fractionally distilled in order to isolate the phenol and acetone components. The composition of typical products at three different operating temperatures (40°, 60°, 80°C) is shown in Table I.

| For Sample #2, collected at 60°C: | |
|---|---|
| Phenol Selectivity, estimated by glc basis cumene hydroperoxide converted: | >99 mole% |
| Phenol selectivity by distillation: | 96.9 mole% |
| Acetone selectivity by distillation: | 95.4 mole% |

TABLE I

| SAMPLE | OPERATING TEMP. (°C) | ← | | | PRODUCT COMPOSITION(WT%) | | | | → |
|---|---|---|---|---|---|---|---|---|---|
| | | ACETONE | CUMENE | PHENOL | STYRENE | ACETO PHENONE | 2-PHENYL 2-PROPANOL | CUMYL PHENOL | CUMENE HYDRO-PEROXIDE |
| 1 | 40 | 34.4 | 12.9 | 47.2 | 1.6 | 0.6 | 1.2 | 0.2 | 1.3 |
| 2 | 60 | 34.6 | 12.8 | 47.5 | 2.0 | 0.8 | 0.7 | 0.3 | 0.4 |
| 3 | 80 | 33.6 | 15.9 | 44.8 | 2.3 | 1.0 | 0.3 | 0.7 | - |

## EXAMPLES 2-18

## PHENOL/ACETONE GENERATION USING ACIDIC ZEOLITE CATALYSTS

Following the procedures, and using the equipment, of Example 1, a series of acidic zeolites were evaluated for phenol/acetone generation using typical crude cumene hydroperoxide feed streams. The product liquid effluents were analyzed by glc and selected samples were fractionally distilled to determine phenol/acetone selectivities. Experimental conditions and glc data for runs made at 40°, 60° and 80°C are summarized in Table II through VI. Of note:

1) In Example 3, an ammonium exchanged Y-zeolite with a silica to alumina ratio of 11:1, CP304-37 from PQ Corporation, 1/16" extrudates, gave:

| | |
|---|---|
| Cumene Hydroperoxide conversion at 40°C of: | >99% |
| Phenol selectivity, by glc: | 94 mole% |
| Phenol selectivity, by distillation: | 94.8 mole% |
| Acetone selectivity, by distillation: | 89.3 moles% |

2) In Example 5, an ammonium exchanged Y-zeolite with a silica to alumina ratio of 3:1, LZY-82 from Union Carbide Corporation, used in extruded form, gave:

| | |
|---|---|
| Cumene hydroperoxide conversion at 80°C of: | 97% |
| Phenol selectivity, by glc: | 99 mole% |
| Phenol selectivity, by distillation: | 93.2 mole% |
| Acetone selectivity, by distillation: | 89.6 mole% |

3) In Example 8, a mordonite with a silica to alumina ratio 10-13:1, Zeolon-900H, from Norton Company, in 0.16 cm diameter extruded form, gave:

| | |
|---|---|
| Cumene hydroperoxide conversion at 80°C of: | 99% |
| Phenol selectivity, by glc: | 95 mole% |
| Acetone selectivity, by glc: | >99 mole% |

4) In Example 9, Valfor C861 $\beta$, a beta zeolite with a silica-to-alumina ratio of 25, containing some 20% alumina binder, in 0.16cm diameter extruded form, gave:

| | |
|---|---|
| Cumene hydroperoxide conversion at 60°C of: | >99% |
| Phenol selectivity, by glc: | 94% |
| Acetone selectivity, by glc: | >99% |
| Phenol selectivity, by distillation: | 94.7% |
| Acetone selectivity, by distillation: | 97.8% |

5) In Example 11, CP316-26, a Y-zeolite with a silica-to-alumina ratio of 46, a unit cell size of 24.26A and a titratable acidity of 0.15 meq/g, gave >99% cumene hydroperoxide conversion at 40°C. Again this catalyst was in extruded form.

6) In Examples 12-14, a series of lanthanum exchanged Y-zeolites with different acidities and unit cell sizes, exhibit different levels of activity in the subject reaction in the order shown below:

| Y-Zeolite | Unit Cell Size (A) | CHP Conversation At 40°C (%) |
|---|---|---|
| LZ-10, LA Exchanged | 24.32 | 97.2 |
| Y-85, LA Exchanged | 24.49 | 87.8 |
| USY, LA Exchanged | 24.56 | 80.5 |

Each catalyst was evaluated in an extruded form.

7) In Examples 15-17, a comparison of Y-zeolite, Y-85, in 0.24 cm diameter extruded form, before and after ammonium fluoride treatment, shows improved performance for the treated catalyst as shown

below:

| Y-Zeolite | EXCHANGE Procedure | CHP Conversion at 40°C (%) |
|---|---|---|
| Y-85 | None | 90 |
| Y-85 | $NH_4F$, 3 hrs, 60°C | 97 |
| Y-85 | $NH_4F$, 6 hrs, 60°C | 96 |

TABLE II

| Example | Catalyst | CHP kg/hr | Recycle (cc/min) | Temp.(°C) | Sample | Acetone | Cumene | Phenol | α-Me Styrene | Aceto-phenone | 2-Ph 2-PrOH | CHP | 4-Cumyl Phenol |
|---|---|---|---|---|---|---|---|---|---|---|---|---|---|
| | | | | | PRODUCT COMPOSITION (%) | | | | | | | | |
| 2 | CP304-37[a] | | | | FS | | | | | | | 78.7 | |
| | | 0.43 | 367 | 40 | 1 | 2.6 | 21.8 | 4.3 | | 0.5 | 5.4 | 65.3 | |
| | | | | 60 | 2 | 20.3 | 20.6 | 30.5 | 0.3 | 0.6 | 1.8 | 25.8 | |
| | CP304-37[b] | | | 80 | 3 | 32.9 | 17.2 | 44.0 | 1.0 | 0.6 | 0.5 | 3.2 | 0.2 |
| 3 | | | | | FS | | | | | | | 78.8 | |
| | | 0.43 | 367 | 40 | 1 | 34.0 | 15.5 | 45.8 | 1.3 | 0.5 | 0.2 | 0.1 | 0.7 |
| | | | | 60 | 2 | 33.6 | 15.5 | 45.1 | 1.6 | 0.7 | 0.1 | | 0.8 |
| | | | | 80 | 3 | 33.6 | 15.3 | 45.4 | 1.2 | 0.5 | 0.1 | | 1.2 |
| 4 | Zeolon-900H[c] | | | | FS | | | | | | | 72.9 | |
| | | 0.43 | 367 | 40 | 1 | 15.1 | 19.9 | 19.0 | | 0.04 | 4.8 | 40.8 | |
| | | | | 60 | 2 | 22.7 | 18.9 | 32.1 | | 0.5 | 2.6 | 23.1 | |
| | | | | 80 | 3 | 32.7 | 19.6 | 34.3 | 1.4 | 0.6 | 1.1 | 9.8 | 0.1 |

[a] 20% CP304-37 and 80% $SiO_2/Al_2O_3$ (75:25), extrudates
[b] 20% CP304-37 and 80% $SiO_2/AL_2O_3$ (16:84), extrudates
[c] $SiO_2/Al_2O_3$ (11:1), extrudates

TABLE III

| Example | Catalyst | CHP kg/hr | Recycle (cc/min) | Temp (°c) | Sample | Acetone | Cumene | Phenol | α-Me Styrene | Aceto-phenone | 2-Ph 2-PrOH | CHP | 4-Cumyl Phenol |
|---|---|---|---|---|---|---|---|---|---|---|---|---|---|
| | | | | | | PRODUCT COMPOSITION (%) | | | | | | | |
| 5 | LZY-82 | | | | FS | | | | | | | 72.9 | |
| | | 0.43 | 367 | 40 | 1 | 34.0 | 15.8 | 42.0 | 2.3 | 0.9 | 0.7 | 2.7 | 0.4 |
| | | | | 60 | 2 | 33.0 | 15.8 | 43.5 | 2.4 | 1.0 | 0.3 | 1.9 | 0.5 |
| | | | | 80 | 3 | 32.3 | 16.1 | 43.3 | 2.9 | 1.0 | 0.3 | 2.2 | 0.5 |
| 6 | LZY-62 | | | | FS | | | | | | | 78.8 | |
| | | 0.43 | 367 | 40 | 1 | 22.6 | 16.4 | 30.6 | 2.3 | 0.8 | 2.0 | 24.9 | |
| | | | | 60 | 2 | 27.9 | 15.3 | 37.4 | 3.0 | 1.0 | 1.1 | 13.7 | 0.1 |
| | | | | 80 | 3 | 31.2 | 14.7 | 41.8 | 3.2 | 0.9 | 0.7 | 7.0 | 0.1 |
| 7 | LZY-72 | | | | FS | | | | | | | 76.2 | |
| | | 0.43 | 367 | 40 | 1 | 8.5 | 15.1 | 11.6 | 0.8 | 0.5 | 5.0 | 58.0 | |
| | | | | 60 | 2 | 24.8 | 13.8 | 33.6 | 2.2 | 0.8 | 1.7 | 22.6 | 0.2 |
| | | | | 80 | 3 | 31.0 | 13.1 | 42.7 | 3.3 | 1.0 | 0.6 | 6.8 | 0.3 |
| 8 | Zeolon-900H[a] | | | | FS | | | | | | | 78.8 | |
| | | 0.43 | 367 | 40 | 2 | 3.5 | 21.4 | 5.0 | 0.4 | 0.5 | 5.8 | 63.4 | |
| | | | | 60 | 4 | 31.8 | 17.4 | 43.5 | 1.0 | 0.6 | 1.7 | 3.7 | 0.1 |
| | | | | 80 | 5 | 33.2 | 17.0 | 45.7 | 1.2 | 0.6 | 0.8 | 1.2 | 0.1 |

[a] $SiO_2/Al_2O_3$(10-13:1), Extrudates

TABLE IV

| | | | | ← Product Composition → | | | | | | | | | |
|---|---|---|---|---|---|---|---|---|---|---|---|---|---|
| Example | Catalyst | CHP kg/hr | Recycle (cc/min) | Temp. (°C) | Sample | Acetone | Cumene | PhOH | β-Me Styrene | Aceto Phenone | 2-Ph 2-PrOH | CHP | DP MP | Cumyl PhOH |
| (9) | Valfor C861β | | | | FS | | | | | | | 78.8 | | |
| | | 0.43 | 367 | 40 | 1 | 33.7 | 15.2 | 45.4 | 3.4 | 0.5 | 0.7 | 0.6 | 0.1 | 0.1 |
| | | | | 60 | 2 | 33.7 | 15.1 | 45.9 | 3.5 | 0.6 | 0.4 | | 0.2 | 0.2 |
| | | | | 80 | 3 | 33.4 | 15.0 | 45.8 | 3.5 | 0.7 | 0.3 | | 0.4 | 0.4 |
| (10) | Valfor CP308-51 | | | 40 | 1 | 2.5 | 21.1 | 2.6 | 0.8 | 0.4 | 6.5 | 66.1 | | |
| | | | | 60 | 2 | 11.6 | 19.9 | 15.0 | 2.0 | 0.5 | 4.0 | 46.9 | | |
| | | | | 80 | 3 | 26.0 | 17.3 | 39.0 | 3.4 | 0.8 | 1.0 | 11.9 | 0.3 | 0.1 |
| (11) | Valfor CP316-26 | | | FS | | | | | | | | 82.6 | | |
| | | | | 40 | 1 | 34.0 | 14.3 | 44.7 | 1.7 | 0.5 | 1.9 | 0.2 | 1.5 | 1.0 |
| | | | | 60 | 2 | 34.4 | 14.3 | 45.0 | 1.2 | 0.5 | 0.5 | | 2.0 | 1.8 |
| | | | | 80 | 3 | 33.6 | 14.6 | 45.3 | 0.6 | 0.6 | 0.4 | | 1.4 | 2.9 |

TABLE V

| Ex. | Catalyst | CHP kg/hr | Recycle (cc/min) | Temp. (°C) | Sample | Acetone | Cumene | PhOH | α-Me Styrene | Aceto-phenone | 2-Ph 2-PrOH | CHP | DP MP | Cumyl PhOH |
|---|---|---|---|---|---|---|---|---|---|---|---|---|---|---|
| | | | | | ← Product Composition → | | | | | | | | | |
| (12) | LZ-10La Exchanged | | | | FS | | | | | | | 78.8 | | |
| | Exchanged | 0.43 | 367 | 40 | 1 | 31.2 | 15.5 | 43.3 | 1.2 | 0.5 | 4.7 | 2.2 | 0.8 | 0.4 |
| | | | | 60 | 2 | 27.9 | 15.1 | 48.6 | 1.4 | 0.7 | 1.4 | 0.1 | 2.2 | 1.5 |
| | | | | 80 | 3 | 30.7 | 15.4 | 46.5 | 1.3 | 0.7 | 0.6 | | 2.5 | 1.7 |
| (13) | LA Exchanged Y-85 | | | | FS | | | | | | | 79.4 | | |
| | | 0.43 | 367 | 40 | 1 | 27.6 | 15.2 | 35.8 | 1.7 | 0.5 | 8.6 | 9.7 | 0.4 | 0.1 |
| | | | | 60 | 2 | 18.6 | 15.2 | 55.6 | 2.0 | 0.7 | 3.6 | 1.9 | 1.6 | 0.5 |
| | | | | 80 | 3 | 34.2 | 14.7 | 44.1 | 1.9 | 0.7 | 0.9 | 0.2 | 2.1 | 0.7 |
| (14) | LA Exchanged US 4 | | | | FS | | | | | | | 80.2 | | |
| | | 0.43 | 367 | 40 | 1 | 24.3 | 14.2 | 32.4 | | 0.5 | 12.2 | 15.6 | 0.4 | 0.2 |
| | | | | 60 | 2 | 31.9 | 13.8 | 41.1 | 1.9 | 0.6 | 4.2 | 4.3 | 1.5 | 0.4 |
| | | | | 80 | 3 | 33.5 | 13.7 | 44.1 | 2.4 | 0.8 | 1.5 | 0.7 | 2.2 | 0.8 |

**TABLE VI**

| Example | Catalyst | CHP kg/hr | Recycle (cc/min) | Temp. (°C) | Sample | Acetone | Cumene | PhOH | α-Me Styrene | Acetophenone | 2-Ph 2-PrOH | CHP | DP MP | Cumyl PhOH |
|---|---|---|---|---|---|---|---|---|---|---|---|---|---|---|
| | | | | | | ← Product Composition → | | | | | | | | |
| (15) | Y-85 | | | | FS | | | | | | | 80.5 | | |
| | | 0.43 | 367 | 40 | 1 | 28.8 | 15.0 | 37.8 | 2.0 | 0.5 | 7.1 | 8.0 | 0.2 | 0.2 |
| | | | | 60 | 2 | 30.4 | 14.7 | 46.4 | 2.4 | 0.7 | 2.3 | 1.5 | 0.8 | 0.3 |
| | | | | 80 | 3 | 30.5 | 14.7 | 47.6 | 2.5 | 0.9 | 1.1 | 0.3 | 1.3 | 0.7 |
| (16) | Y-85,$NH_4F$,3 | | | | FS | | | | | | | 80.5 | | |
| | | 0.43 | 367 | 40 | 1 | 31.2 | 14.5 | 44.1 | 1.7 | 0.5 | 4.6 | 2.4 | 0.6 | 0.2 |
| | | | | 60 | 2 | 33.9 | 14.1 | 45.6 | 1.8 | 0.5 | 1.3 | 0.3 | 1.6 | 0.7 |
| | | | | 80 | 3 | 34.4 | 14.0 | 45.3 | 1.7 | 0.6 | 0.6 | | 2.0 | 1.0 |
| (17) | Y-85,$NH_4F$,6 | | | | FS | | | | | | | 82.5 | | |
| | | 0.43 | 367 | 40 | 1 | 32.4 | 14.8 | 42.3 | 1.9 | 0.5 | 4.4 | 2.9 | 0.4 | 0.2 |
| | | | | 60 | 2 | 34.9 | 14.3 | 44.7 | 1.9 | 0.5 | 1.5 | 0.3 | 1.1 | 0.5 |
| | | | | 80 | 3 | 34.6 | 14.2 | 44.9 | 2.0 | 0.6 | 0.6 | | 1.7 | 1.0 |
| (18) | LZ-20 | | | | FS | | | | | | | | | |
| | | | | 40 | 1 | 31.3 | 14.7 | 43.2 | 1.9 | 0.5 | 4.5 | 2.7 | 0.7 | 0.2 |
| | | | | 60 | 2 | 34.0 | 14.4 | 45.0 | 2.2 | 0.5 | 1.3 | 0.3 | 1.5 | 0.5 |
| | | | | 80 | 3 | 33.3 | 14.6 | 45.4 | 2.4 | 0.6 | 0.7 | 0.1 | 1.8 | 0.7 |

## Claims

1. A method for synthesis of phenol and acetone from cumene hydroperoxide which comprises decomposing cumene hydroperoxide using as a catalyst a crystalline aluminosilicate mordonite-type zeolite or faujasite-type zeolite selected from Y-zeolites, ammonium exchanged, thermally-stabilized, dealuminated Y-zeolites, rare-earth exchanged Y-zeolites and fluoride-treated Y-zeolites.

2. A method as claimed in Claim 1 wherein said dealuminated Y-seolite has a silica-to-alumina ratio of greater than 5:1.

3. A method as claimed in Claim 1 or Claim 2 wherein said Y-zeolite has a titratable acidity up to 1.0 meg/g.

4. A method as claimed in any one of the preceding claims wherein the catalyst is in the form of extrudates, granules, tablets or spheres.

5. A method as claimed in any one of the preceding claims wherein the Y-zeolite is treated with ammonium fluoride.

6. A method as claimed in any one of the preceding claims which further comprises using a recycle loop reactor connected in series with a finishing reactor.

7. A method as claimed in any one of the preceding claims wherein the reaction temperature is from 20°C to 150°C.

8. The method of Claim 1 wherein the reaction pressure is from about 690 kPa gauge to about 6900 kPa gauge.

9. A method as claimed in any one of the preceding claims wherein the end product is fractionally distilled.

10. A method as claimed in any one of the preceding claims wherein the cumene hydroperoxide is about 80% pure.

FIG.1

RECYCLE LOOP REACTOR SYSTEM